# EUROPEAN PATENT SPECIFICATION

(11) **EP 2 470 166 B1**
(45) Date of publication and mention of the grant of the patent: **14.08.2013**
(21) Application number: 10805414.9
(22) Date of filing: 23.08.2010
(51) Int. Cl.: A61K 9/16, A61K 9/20, A61K 9/28, A61K 9/50

(54) **NEW COMPOSITIONS OF 1-[2-(2,4-DIMETHYL-PHENYLSULFANYL)-PHENYL]PIPERAZINE**
NEUE ZUSAMMENSETZUNGEN AUS 1-[2-(2,4-DIMETHYL-PHENYLSULFANYL-)PHENYL-]PIPERAZIN
NOUVELLES COMPOSITIONS DE 1-[2-(2,4-DIMÉTHYL-PHÉNYLSULFANYL)-PHÉNYL]PIPÉRAZINE

(30) Priority: 24.08.2009 DK 200900950; 24.08.2009 US 236223 P
(43) Date of publication of application: 04.07.2012
(73) Proprietor: H. Lundbeck A/S, 2500 Valby (DK)
(72) Inventor: HØJER, Astrid-Maria, DK-2830 Virum (DK); DREWES, Pernille, Gundorf, DK-2830 Virum (DK); KATEB, Jens, S-21743 Malmö (SE)
(74) Representative: Conrad, Lars Sparre
(86) International application number: PCT/DK2010/050216
(87) International publication number: WO 2011/023194

(56) References cited:
- WO-A1-2007/144005
- HELLSTROM W.J.G.: "Emerging treatments for premature ejaculation: focus on dapoxetine", NEUROPSYCHIATRIC DISEASE AND TREATMENT, vol. 5, 8 April 2009 (2009-04-08), pages 37-46, XP002660112,
- GOLDEN R.N. ET AL.: "Efficacy and tolerability of controlled-release and immediate-release paroxetine in the treatment of depression", THE JOURNAL OF CLINICAL PSYCHIATRY UNITED STATES, vol. 63, no. 7, July 2002 (2002-07), pages 577-584, XP009152606,

## Description

### Field of the invention

The present invention relates to pharmaceutical compositions comprising 1-[2-(2,4-dimethyl-phenylsulfanyl)-phenyl]piperazine and pharmaceutically acceptable acid addition salts thereof.

### Background

The compound 1-[2-(2,4-dimethyl-phenylsulfanyl)-phenyl]piperazine was first disclosed in the international patent application published as WO 03/029232. Subsequent applications WO 2007/144005, WO 2008/113359 and WO 2009/062517 disclose additional uses, pharmaceutical compositions and processes of manufacture. From hereon, 1-[2-(2,4-dimethyl-phenylsulfanyl)-phenyl]piperazine and pharmaceutically acceptable acid addition salts thereof is referred to as "Compound I" or "Compound I XX" when reference to the specific XX salt of Compound I is desired. Compound I has been shown to have a unique pharmacological profile being a 5HT enhancer with antagonist effects at 5HT₃ receptors (Kᵢ: 4.5nM), agonist effects at 5HT_{1A} receptors (EC₅₀: 200nM) and inhibitory effects on the serotonin transporter (IC₅₀: 5.4nM). [Soc.Biol.Psych. 64th annual meeting, May 14-16, 2009 Vancouver, Canada, Poster #260]. Additionally, Compound I has in non-clinical *in vivo* studies shown to produce significant increases in acetylcholine, norepinephrine, dopamine, and serotonin in brain regions associated with mood regulation. [World Federation of Societies of Biological Psychiatry, 9th World Congress of Biological Psychiatry, 28.June-2.July, 2009, Paris, France, Poster P-29-005]. Based on the pharmacological profile, Compound I is believed to be effective in the treatment of mood disorders, such as depression, anxiety, and also useful in the treatment of cognitive impairment and pain. This notion appears to be supported by the first clinical trial reported for Compound I which shows that Compound I dosed at 5 and 10 mg is safe and well-tolerated and efficacious in the treatment of major depressive disorder [American Psychiatric Association, 162nd Annual Meeting, 16-21 May, 2009, San Fransisco, USA, Poster NR4-024].

Treatment of depressed patients with anti-depressants, such as selective serotonin re-uptake inhibitors (SSRI) or serotonin and noradrenaline re-uptake inhibitors (SNRI) are generally associated with adverse events, such as nausea, sleep disturbances, sexual dysfunction, weight gain, headache and dry mouth [Int.J.Psych.Clin.Pract., 10, 31-37, 2006]. Lowering the amount or severity of adverse events associated with treatment with anti-depressants is an obvious desire as it will increase patient comfort, increase compliance and thus ultimately improve treatment outcome.

Treatment of depression with the SSRI paroxetine administered as an immediate release (IR) tablet and as an enteric coated, sustained release tablet has been compared [J.Clin Psych., 63, 577-584, 2002]. Treatment with enteric coated tablets was associated with less nausea. Other types of gastro intestinal (GI) related adverse events, such as diarrhea, however, appeared to increase as did the number of reported abnormal ejaculation, female genital disorders and dizziness.

### Summary of the invention

The present inventors have found that administering 1-[2-(2,4-dimethyl-phenylsulfanyl)-phenyl]piperazine and pharmaceutically acceptable acid addition salts thereof (Compound I) in a way so that Compound I is not released in the stomach lowers the amount of adverse events, and in particular the amount of GI tract related adverse events. Accordingly, in one embodiment, the invention relates to a pharmaceutical composition for oral administration comprising 1-[2-(2,4-dimethyl-phenylsulfanyl)-phenyl]piperazine and pharmaceutically acceptable acid addition salts thereof as active compound wherein said composition is adapted so that release of said active compound does not take place in the stomach.

In one embodiment, the invention relates to a method of treating a disease, said method comprising the administration of a pharmaceutical composition of the present invention to a patient in need thereof.

In one embodiment, the invention relates to the use of the compound 1-[2-(2,4-dimethyl-phenylsulfanyl)-phenyl]piperazine and pharmaceutically acceptable acid addition salts thereof in the manufacture of a medicament for oral administration for the treatment of a disease, wherein said medicament is adapted so that the release of said compound does not take place in the stomach.

In one embodiment, the invention relates to the compound 1-[2-(2,4-dimethyl-phenylsulfanyl)-phenyl]piperazine and pharmaceutically acceptable acid addition salts thereof for use in the treatment of a disease, wherein said compound is in a pharmaceutical composition for oral administration adapted so that release of said compound does not take place in the stomach.

### Figures

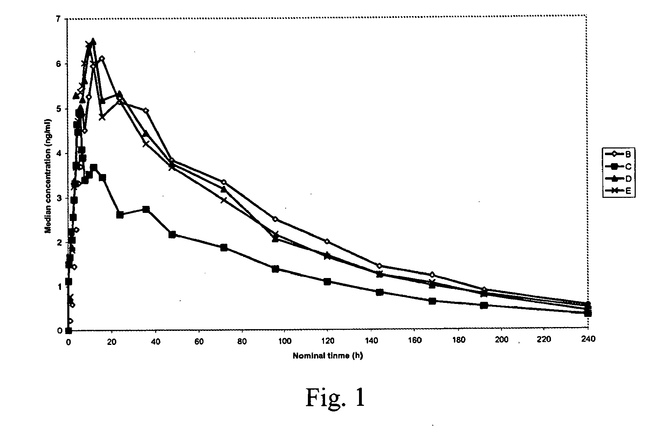

Figure 1: Plasma concentration-time profiles obtained upon administration of compound I HBr. B: 20 mg compound I HBr IR; C: 9 mg Compound I HBr iv; D: 20 mg Compound I HBr released in the proximal bowl; E: 20 mg Compound I HBr released in the distal bowl.

### Detailed description of the invention

In one embodiment, the invention relates to a pharmaceutical composition for oral administration comprising 1-[2-(2,4-dimethyl-phenylsulfanyl)-phenyl]piperazine and pharmaceutically acceptable acid addition salts thereof as active compound wherein said composition is adapted so that said active compound is not released in the stomach. In particular, Compound I is released in the intestine and in particular in the small intestine.

In the present context, "not released in the stomach" is intended to indicate that Compound I upon administration to a subject is substantially not present in the stomach in dissolved form.

The presence of Compound I in the stomach in dissolved form may in principle be determined *in vivo* using techniques such as X-ray evaluation, NMR imaging, gamma scintigraphy, or indeed, direct sampling from the stomach. These tests are, however, difficult to carry out in humans. A more convenient test to determine the presence of Compound I in the stomach is a two-stage *in vitro* dissolution test, which incorporates a first phase in which the pharmaceutical composition is exposed to low pH mimicking the environment in the stomach, followed by a second stage with exposure to higher pH mimicking the environment in the small intestine. The assay criteria are guided by the pH in various parts of the GI tract and the time it takes e.g. a tablet or a capsule to pass through the stomach and into the intestine, as discussed below.

A useful two-stage *in vitro* dissolution test is as follows. Equipment: Standard USP rotating paddle apparatus; paddle speed 75 rpm; 37 °C. First stage: A unit dose is exposed to 600 ml 0.1 M HCl for 2 hours; second stage: The unit dose is transferred to 900 ml TRIS buffer (0.6 M), pH=6.8. A unit dose typically comprises 1-50 mg Compound I, such as 10, 20 or 30 mg of Compound I. Throughout this application, reference to a certain amount of compound I (e.g. 1-50 mg) is to be understood as referring to an amount of Compound I corresponding to said certain amount of the free base.

In one embodiment, a pharmaceutical composition for oral administration comprising Compound I is said to be adapted so that compound I is not released in the stomach if less than 30%, such as less than 20%, such as less than 10% of compound I is released in stage 1 as defined above. In a further embodiment, a pharmaceutical composition for oral administration is said to be adapted so that Compound I is not released in the stomach if it, in addition to the criterion above, releases at least 20%, such as at least 30%, such as at least 50% in stage 2 as defined above after 3 hours. In a further embodiment, a pharmaceutical composition for oral administration comprising Compound I is said to be adapted so that compound I is not released in the stomach if it, in addition to the criteria above, releases at least 60%, such as at least 70%, such as at least 80% of compound I in stage 2 as defined above after 5 hours. In a further embodiment, a pharmaceutical composition for oral administration comprising Compound I is said to be adapted so that compound I is not released in the stomach if it, in addition to the criteria above, releases at least 80%, such as at least 90%, such as at least 95% of compound I in stage 2 as defined above after 8 hours.

In a particular embodiment, a pharmaceutical composition for oral administration comprising Compound I is said to be adapted so that compound I is not released in the stomach if less than 30% of compound I is released in stage 1 as defined above, and at least 20% released in stage 2 as defined above after 3 hours, and at least 60% of compound I is released in stage 2 as defined above after 5 hours, and at least 80% of compound I is released in stage 2 as defined above after 8 hours.

In a particular embodiment, a pharmaceutical composition for oral administration comprising Compound I is said to be adapted so that compound I is not released in the stomach if less than 10% of compound I is released in stage 1 as defined above, and at least 60% released in stage 2 as defined above after 1 hours, and at least 80% of compound I is released in stage 2 as defined above after 2 hours, and at least 95% of compound I is released in stage 2 as defined above after 3 hours. The dissolution time and amount released in stage 1 are included in the dissolution time and amount released in stage 2 in the assay criteria above.

The examples provide an assay for analysing Compound I.

In humans, the GI tract comprises i.a. the stomach, the small intestine and the large intestine. The stomach is connected to the throat via the oesophagus, and in the stomach the food is churned before it is moved to the small intestine. The stomach may hold 1 - 1.5 litre of food. Importantly in the present context, pH in the stomach is low, i.e. around 1-2. The small intestine comprises three compartments, i.e. duodenum, jejunum and ileum together measuring up to 7 meters in length and with a diameter of 2.5-3 cm. The major part of the chemical digestion of the food takes place in the small intestine. Digestive enzymes, such as proteases, lipases and amylases hydrolyse the food to amino acids, fatty acids and glycerol, and mono saccharides (e.g. glucose). These nutrients then pass through the wall of the small intestine into the blood. The major part of the nutrients from food is absorbed from the small intestine. The pH of the small intestine is markedly higher than in the stomach, i.e. around 5.5 or above. The large intestine is about 1.5 meter long and from here water is absorbed from the food and faeces is compacted and stored in the rectum before elimination through anus. Bacteria present in the large intestine create vitamins, e.g. vitamin B and K which are also absorbed. The pH in the large intestine increases from around 5.5 to 7.

In the fasted state, indigestible non-disintegrating solids are emptied from the stomach during phase III of the Interdigestive Migrating Myoelectric Complex (IMMC), which occurs approximately every 2 hours in humans. Depending on the stage of the IMMC at the time of dosing in the fasted state, a tablet or a capsule may exit the stomach almost immediately after dosing, or as long as 2 hr after dosing. In the fed state, a small tablet or capsule will empty slowly from the stomach with the contents of the meal. Larger tablets or capsules will be retained in the stomach for the duration of the digestion of the meal, and will exit into the duodenum during phase III of an IMMC, after the entire meal has been digested and has exited the stomach.

The structure of Compound I is depicted below 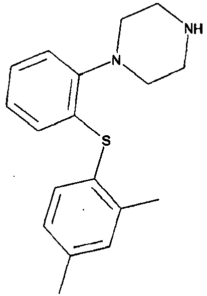 In one embodiment, said pharmaceutically acceptable acid addition salts are salts of acids that are non-toxic. Said salts include salts made from organic acids, such as maleic, fumaric, benzoic, ascorbic, succinic, oxalic, bis-methylenesalicylic, methanesulfonic, ethanedisulfonic, acetic, propionic, tartaric, salicylic, citric, gluconic, lactic, maleic, mandelic, cinnamic, citraconic, aspartic, stearic, palmitic, itaconic, glycolic, p-arninobenzoic, glutamic, benzenesulfonic, theophylline acetic acids, as well as the 8-halotheophyllines, for example 8-bromotheophylline. Said salts may also be made from inorganic salts, such as hydrochloric, hydrobromic, sulfuric, sulfamic, phosphoric and nitric acids. Particular mention is made of salts made from methanesulfonic acid, maleic acid, fumaric acid, meso-tartaric acid, (+)-tartaric acid, (-)-tartaric acid, hydrochloric acid, hydrobromic acid, sulphuric acid, phosphorous acid and nitric acid. Distinct mention is made of the hydrobromide salt.

Processes for the manufacture of Compound I are known in the art. For example, WO 03/032292 and WO 2007/144005 disclose useful synthetic routes. Pharmaceutical compositions comprising Compound I are known in the art. WO 2007/144005 (page 16) discloses that Compound I may be administered orally in the form of tablets or parenterally in the form of solutions for injections. Nothing, however, is disclosed on the relationship between administration form and adverse events profile.

In one embodiment, the pharmaceutical composition of the present invention is a sustained release composition. In the present context, "sustained release" is intended to indicate that Compound I is slowly released from said composition.

In one embodiment, the pharmaceutical composition of the present invention is a delayed release composition. In the present context "delayed release" is intended to indicate that little or no Compound I is released from said composition for a predetermined time after which Compound I is released either quickly (immediate release, IR) or in a sustained fashion.

Sustained release compositions are known in the art, and the following provides examples of how to provide a sustained release composition comprising Compound I. Specific examples are also provided in the Examples part.

In one embodiment, a sustained release composition is achieved by means of a matrix composition in which Compound I is embedded or dispersed in a matrix which retards the release of Compound I into an aqueous medium, e.g. the fluid of the GI tract. Compound I is released mainly from the surface of said matrix after diffusion through the matrix. Alternatively, the matrix slowly erodes exposing fresh surface from which Compound I is released. In some matrixes, both mechanisms operate simultaneously. Rate of release from a matrix depends i.a. on the matrix particle size. Small particles will give rise to a fast release of Compound I, whereas a large particles will give rise to a slow release of Compound I. The rate of release also depends on the matrix material, i.e. the diffusion coefficient of Compound I in said matrix material. Generally, polymers such as cellulose acetate have low diffusion coefficients while hydrogels have high diffusion coefficients. Thus, by selecting matrix material and controlling matrix particle size, it is possible to control the release rate of Compound I. Additionally, plasticizers, pores and pore-inducing additives may be used to manipulate release rates.

In one embodiment, the pharmaceutical composition of the present invention is a multiparticulate matrix composition, i.e. a composition comprising a plurality a particles comprising Compound I in a matrix which retards the release of Compound I. Particles in a multiparticulate composition typically have diameters in the range of 50 µm to 3 mm. Matrix material useful for this embodiment includes microcrystalline cellulose such as Avicel, including grades of microcrystalline cellulose to which binders such as hydroxypropyl methyl cellulose have been added, waxes such as paraffin, modified vegetable oils, carnauba wax, hydrogenated castor oil, beeswax, polyvinyl chloride, polyvinyl acetate, copolymers of vinyl acetate and ethylene, and polystyrene. Water soluble binders or release modifying agents which can optionally be formulated into the matrix include water-soluble polymers such as hydroxypropyl cellulose (HPC), hydroxypropyl methyl cellulose (HPMC), methyl cellulose, PVP, poly(ethylene oxide) (PEO), PVA, xanthan gum, carrageenan. In addition, materials which function as release-modifying agents include water-soluble materials such as sugars or salts.

Multiparticulate matrix compositions may be prepared by extrusion/spheronization, in which Compound I is wet-massed with a binder, extruded through a perforated plate and finally placed on a rotating disc which breaks the extrudates into rounded spheres. Alternatively, said composition is wax granulates. Wax granulates may be prepared by dissolving Compound I in liquid wax, which upon cooling (and solidification) is forced through a screen to form granules. Suitable waxes include hydrogenated castor oil, camuba wax and stearyl alcohol. If the melting point of the wax is too high, the wax and Compound I may be mixed in an organic solvent to form a paste, which is forced through a screen to form granules. Once formed, particles of the multiparticulate matrix composition may be mixed with e.g. lactose, microcrystalline cellulose or dicalcium phosphate and compressed into tablets. Disintegrants may also be applied. Upon administration of such tablet, it disintegrates when exposed to aqueous medium, such as in the GI tract, exposing a multiparticulate matrix composition comprising Compound I, which compound is then slowly released. Alternatively, particles of the multiparticulate matrix composition may be administered in a capsule, in a sachet or as a powder.

In one embodiment, said matrix composition comprises Compound I and a hydrophilic polymer. This embodiment also includes multiparticulate matrix compositions. Suitable polymers include hydroxypropylmethyl cellulose (HPMC), hydroxypropyl cellulose (HPC), poly (ethylene oxide), PVA, xanthan gum, carbomer, carrageenan, and zooglan. Upon administration, the hydrophilic polymer is swollen by, and eventually dissolves in, aqueous medium, e.g. in the GI tract. Compound I is released both by diffusion from the matrix and by erosion of the matrix. The rate of release of Compound I may be controlled by the amount and molecular weight of hydrophilic polymer employed. In general, using a greater amount of the hydrophilic polymer decreases the dissolution rate, as does using a higher molecular weight polymer. Using a lower molecular weight polymer increases the dissolution rate. The dissolution rate may also be controlled by the use of water-soluble additives such as sugars, salts, or soluble polymers. Examples of these additives are sugars such as lactose, sucrose, or mannitol, salts such as NaCl, KCI, NaHCO₃, and water soluble polymers such as PNVP or PVP, low molecular weight HPC or HMPC or methyl cellulose.

In one embodiment, the matrix composition comprises Compound I dispersed in a hydrogel. Hygrogels are water-swellable network polymers. As discussed above, hydrogels are characterised by relatively high diffusion coefficients, which allows the preparation of relatively large dosage forms, e.g. tablets rather than multiparticulate matrixes. Hydrogel tablets may be prepared and sold as swoolen gels, or alternatively prepared and sold in a dry, non-swollen form.

In one embodiment, and as an alternative to matrix compositions, the invention provides a sustained release composition which is a membrane-moderated composition, in which a reservoir of Compound I is coated by a membrane, which membrane effects a retarding of the release of Compound I. Such composition form may be large, e.g. membrane coated tablets, or small, e.g. membrane coated particles to be presented e.g. in a capsule, as a powder or in a sachet, or compressed into tablets. Sustained release coatings include polymer coatings, such as ethyl cellulose, cellulose acetate and cellulose acetate butyrate. The polymer may be applied as a solution in an organic solvent or as an aqueous dispersion or latex. The coating operation may be conducted in standard equipment such as a fluid bed coater, a Wurster coater, or a rotary bed coater.

In one embodiment, the composition of the present invention is a capsule comprising Compound I, said capsule having a shell comprising a membrane, which membrane effects a retarding of the release of Compound I.

In one embodiment, a multiparticulate composition of the present invention is prepared by applying Compound I on an inert core by drug-layering techniques, such as powder-coating or by spraying a solution of Compound I and a suitable binder onto a core, e.g. in a fluidized bed coater or a rotary mixer. Alternatively, cores comprising Compound I may be prepared by extrusion/spheronization process described above, or granulation e.g. in a fluid bed. Further details on preparation of tablets (or cores) are provided below. The particles achieved are subsequently coated with a suitable membrane which retards the release of Compound I.

A sustained release composition of the present invention may also be achieved using osmotic delivery systems. Such system comprises a core containing an osmotically effective composition and Compound I surrounded (fully or partly) by a semi-permeable membrane. Water, but not solutes dissolved in the water can pass through a semi-permeable membrane. When placed in an aqueous environment (e.g. the GI tract), the core absorbs water which generates an increased pressure inside the delivery system. This increased pressure will drive Compound I out of the delivery system, e.g. through a pre-manufactured opening. Alternatively, the delivery system may burst once the pressure has reached a certain level. Suitable osmotically effective compounds include salts, sugar, and water-swellable polymers. Materials useful for semi-permeable membranes include polyamides, polyesters and cellulose derivatives, such as cellulose acetate, cellulose acetate butyrate and ethyl cellulose.

As mentioned above, a pharmaceutical composition of the present invention may also be achieved by means of a delayed release composition. The following provides examples of how delayed release compositions may be prepared. Specific examples are provided in the Examples part.

In one embodiment, the delay is achieved by means of a pH sensitive coating. In particular, said coating substantially remains intact, e.g. does not dissolve or disintegrate, at the pH found in the stomach, thereby substantially preventing release of Compound I. Moreover, said coating disintegrates or dissolves (or similar) at the higher pH found in the intestine, such as the small intestine, allowing release of compound I.

In one embodiment, the composition of the present invention is coated with a pH sensitive coating which substantially only allows Compound I to be released in the small intestine. Such composition is often referred to as an enteric composition, and similarly the coating is referred to as an enteric coating.

pH sensitive coatings include pH sensitive polymers, such as polyacrylamides, phthalate derivatives such as acid phthalates of carbohydrates, amylose acetate phthalate, cellulose acetate phthalate, other cellulose ester phthalates, cellulose ether phthalates, hydroxypropyl cellulose phthalate, hydroxypropylethyl cellulose phthalate, hydroxypropylmethyl cellulose phthalate, methylcellulose phthalate, polyvinyl acetate phthalate, polyvinyl acetate hydrogen phthalate, sodium cellulose acetate phthalate, starch acid phthalate, styrene-maleic acid dibutyl phthalate copolymer, styrene-maleic acid polyvinylacetate phthalate copolymer, styrene and maleic acid copolymers, polyacrylic acid derivatives such as acrylic acid and acrylic ester copolymers, polymethacrylic acid and esters thereof, poly acrylic methacrylic acid copolymers, shellac, and vinyl acetate and crotonic acid copolymers.

Anionic acrylic copolymers of methacrylic acid and methylmethacrylate are particularly useful pH dependent coating materials. Enteric coatings of this type are available from degussa under the tradename Eudragit. Particularly useful are the products Eudragit L, which comprise methacrylic acid-ethyl acrylate copolymer (1:1), and Eudragit S which comprises methacrylic acid-ethyl acrylate copolymer (1:2). Eudragit L dissolves around pH 5.5 and Eudragit S dissolves at pH around 7. Thus, by applying either of the Eudragit polymers in pure form or as mixtures thereof, it is possible to control where in the intestine release takes place.

In one embodiment, the invention provides a multiparticulate composition, wherein each particle is coated with a pH sensitive coating, such as an enteric coating. These particles may be prepared by applying Compound I on an inert core by drug-layering techniques, such as powder-coating, or by spraying a solution of Compound I and a suitable binder onto a core, e.g. in a fluidized bed coater or a rotary mixer. Alternatively, particles with Compound I dispersed therein may be prepared as described above. The particles achieved are subsequently coated with a suitable pH sensitive coating, e.g. en enteric coating. These particles may be compressed into a tablet, as described above, or presented in a capsule, as a powder or in a sachet.

In one embodiment, the invention provides a tablet coated with a pH sensitive coating, e.g. an enteric coating. The tablet may be prepared in a number of ways available to the skilled person. Tablets may be prepared by mixing Compound I with ordinary adjuvants and/or diluents and subsequently compressing the mixture in a conventional tabletting machine. Examples of adjuvants or diluents include PVP, PVP-VA co-polymers, microcrystalline cellulose, sodium starch glycolate, corn starch, mannitol, potato starch, talcum, magnesium stearate, gelatine, lactose, gums, and the like. Any other adjuvants or additives usually used for purposes such as colourings, flavourings, preservatives etc. may be used provided that they are compatible with the active ingredients.

Alternatively, tablets comprising Compound I may conveniently be prepared by wet granulation. Using this method, the dry solids (active ingredients, filler, binder etc.) are blended and moistened with water or another wetting agent (e.g. an alcohol) and agglomerates or granules are built up of the moistened solids. Wet massing is continued until a desired homogenous particle size has been achieved whereupon the granulated product is dried. Compound I is typically mixed with lactose monohydrate, corn starch and copovidone in a high shear mixer together with water. Following formation of granulates, these granulates may be sieved in a sieve with a suitable sieve size, and dried. The resulting, dried granulates are then mixed with e.g. microcrystalline cellulose, croscarmellose sodium and magnesium stearate, following which the tablets are pressed. Alternatively, tablets can be made by mixing and granulating Compound I in a fluid bed together with suitable excipients, such as mannitol, microcrystalline cellulose, sodium starch glycolate, hydroxypropyl cellulose and magnesium stearate. The granulates obtained are then pressed into tablets.

The tablets obtained are subsequently coated with a suitable pH sensitive coating, e.g. an enteric coating, e.g. by spraying a solution comprising the coating material onto the tablets.

In one embodiment, the invention provides a delayed release composition comprising Compound I, mannitol, microcrystalline cellulose, sodium starch glycolate, hydroxypropyl cellulose and magnesium stearate in a tablet, which tablet is coated with methacrylic acid ethyl acrylate (1:1) polymer, e.g. with a M_{w} around 250,000.

It may be desirable to apply a sub-coat between the core comprising Compound I and the pH sensitive coating, e.g. the enteric coating. Such sub-coat may be desirable for instance if Compound I and the compounds in the pH sensitive coating react thereby compromising the stability of the pharmaceutical composition. Examples of sub-coat material include polyethylene glycol, polyvinyl pyrrolidone, polyvinyl alcohol, hydroxypropyl cellulose and hydroxymethyl cellulose.

As an alternative to a pH sensitive coating, an osmotic delivery system as described above may be used. By choice of membrane, system geometry and osmotically active compound is is possible to delay the osmotic burst until the system has passed through the stomach.

Alternatively, a delayed release composition may be achieved using a core comprising Compound I and a swellable material, such as a hydrogel, which core is coated with a semipermeable membrane. Suitable membranes and hydrogels are discussed above. Upon administration into an aqueous media (e.g. the GI tract), water passes through the membrane, causing the hydrogel to swell. By appropriate choice of membrane material, hydrogel and geometry, it is possible to delay the burst until the core has passed through the stomach.

The plasma concentration-time profile obtained with administration of Compound I in an IR composition is characterised by a relative late tₘₐₓ and a plasma concentration plateau around tₘₐₓ. Such profile is believed to be beneficial because it gives rise to less fluctuation in the plasma concentration in the steady-state, multiple-dose situation, i.e. the situation experienced by the patients. Many adverse events are Cₘₐₓ driven, and the low level of in particular sleep and sexually related adverse events observed in the clinical trials with Compound I (see below) is believed to be associated with this profile. The therapeutic effect of a compound is also dependent on the plasma concentration-time profile it gives rise to. Thus, two administration forms giving rise to the same plasma concentration-time profile would be expected to have the same therapeutic effect. It is unexpected that Compound I when released in the small intestine and, in deed, administered iv, retains the beneficial plasma concentration-time profile characteristics of the IR composition. It is believed that due to the retained plasma concentration-time profile, administration of a pharmaceutical composition of the present invention will give rise to a low level of GI related adverse events while maintaining a low level of sleep and sexually related adverse events and maintaining the beneficial therapeutic effect. Thus, if the dose of Compound I is maintained (as compared to administration in IR tablet) a pharmaceutical composition of the present invention is expected to provide the same therapeutic benefits, however at a markedly lower level of adverse events. On the other hand, the dose may be increased which would be expected to provide an improved therapeutic benefit while maintaining an acceptable level of adverse events. Rephrased, a pharmaceutical composition of the present invention increases the therapeutic window (i.e. the dose of a drug between the amount that gives a therapeutic effect and the amount that gives an unacceptable level of adverse events) for Compound I.

Most pharmaceutical developments apply IR compositions in the initial trails and experiments for convenience and simplicity. From a regulatory perspective it is an added benefit of the compositions of the present invention that they maintain the plasma concentration-time profile of IR compositions. Due to this bioequivalence, it is possible to use data obtained in early studies using an IR composition in support for an application for marketing approval for a composition of the present invention.

As discussed above, Compound I has been shown to have a unique pharmacological profile being a 5HT enhancer, an inhibitor of the serotonin receptor 3 (5-HT₃ antagonist), an agonist of the serotonin receptor 1_{A} (5-HT_{1A} agonist) and an inhibitor of serotonin reuptake. Additionally, Compound I gives rise to an increase in the extracellular levels of serotonin, noradrenaline, dopamine and acetylcholine in rat brains. The International application WO 2008/113359 also discloses the results from clinical trials in depressed patients with Compound I which shows a surprisingly low level of sleep and sexually related adverse events.

On this background, Compound I is expected to be useful in the treatment of mood disorders, such as major depressive disorder, generalised anxiety disorder, panic disorder, post traumatic stress disorder, and depression associated with anxiety, i.e. co-existing depression and anxiety. The impact on extracellular acetylcholine levels is expected to translate into an effect on cognition, cf. the use of acetylcholine esterase inhibitors in the treatment of Alzheimer's disease. Thus, Compound I may also be used in the treatment of depression associated with cognitive impairment and Alzheimer's disease.

A fraction of patients with major depressive disorder will respond to treatment with e.g. a selective serotonin transport inhibitor in the sense that they will improve on clinically relevant scales, such as HAMD or MADRS, but where other symptoms, such as cognitive and/or sleep symptoms remain. In the present context, these patients are referred to as suffering form depression with residual symptoms. Compound I is expected to be useful in the treatment of such patients.

Pre-clinical data presented e.g. in WO 2008/113359 supports the notion that compound I may be used in the treatment of pain. In one embodiment, pain is chronic pain including phantom limb pain, neuropathic pain, diabetic neuropathy, post-herpetic neuralgia (PHN), carpal tunnel syndrome (CTS), HIV neuropathy, complex regional pain syndrome (CPRS), trigeminus neuralgia, tic douloureux, surgical intervention (e.g. post-operative analgesics), diabetic vasculopathy, capillary resistance, diabetic symptoms associated with insulitis, pain associated with menstruation, pain associated with cancer, dental pain, headache, migraine, tension-type headache, trigeminal neuralgia, temporomandibular joint syndrome, myofascial pain, muscular injury, fibromyalgia syndrome, bone and joint pain (osteoarthritis), rheumatoid arthritis, rheumatoid arthritis and edema resulting from trauma associated with bums, strains or fracture bone pain due to osteoarthritis, osteoporosis, bone metastases or unknown reasons, gout, fibrositis, myofascial pain, thoracic outlet syndromes, upper back pain or lower back pain (wherein the back pain results from systematic, regional, or primary spine disease (radiculopathy), pelvic pain, cardiac chest pain, non-cardiac chest pain, spinal cord injury (SCI)-associated pain, central post-stroke pain, cancer neuropathy, AIDS pain, sickle cell pain or geriatric pain. In one embodiment, pain is irritable bowl syndrome (IBS) or fibromyalgia.

On the basis of the pharmacological profile, it is also expected that Compound I may be useful in the treatment of eating disorders, such as obesity, binge eating, anorexia and bulimia nervosa, and substance abuse, such as alcohol, nicotine and drug abuse.

Hence, in one embodiment, the invention relates to a method of treating a disease selected from mood disorders; major depressive disorder; general anxiety disorder; panic disorder; post traumatic stress disorder; depression associated with cognitive impairments, Alzheimer's disease or anxiety; depression with residual symptoms; chronic pain; eating disorder or abuse said method comprising the administration of a therapeutically effective amount of a composition of the present invention to a patient in need thereof. In one embodiment, said composition is a tablet or a multiparticulate composition coated with a pH sensitive coating, such as an enteric coating. In one embodiment, said composition, and in particular said tablet or multiparticulate composition, is adapted so that release of Compound I takes place in the small intestine.

Due to the very low level of sleep, sexually and GI related adverse events observed in treatment with Compound I, the composition of the present invention may also be useful as second line treatment for patients who cannot use other drugs, such as other anti-depressants, such as selective serotonin reuptake inhibitors (SSRI), selective noradrenalin reuptake inhibitors (NRI), noradrenaline/serotonin reuptake inhibitors (SNRI) or tri-cyclics (TCA) due to sleep, sexually or GI related adverse events. In this embodiment, the patient to be treated has received another medication (or is still receiving it), which medication was ceased or reduced (or has to be ceased or reduced) due to sleep, sexually or GI related adverse events.

In one embodiment, the patient to be treated has been diagnosed with the disease said patient is being treated for.

A typical oral dosage is in the range of from about 0.01 to about 5 mg/kg body weight per day, preferably from about 0.01 to about 1 mg/kg body weight per day, administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

Due to the low level of GI related adverse events when using a composition of the present invention, a patient may receive an elevated amount of Compound I, thereby increasing the therapeutic effect while keeping the level of adverse events at an acceptable level. A typical oral dosage for adults is in the range of 5-50 mg/day of Compound I, such as 5-40 mg/day. This may typically be achieved by the administration of 5-50 mg, such as 10-30 mg, such as 5, 10, 15, 20 25, 30 or 40 mg of Compound I once or twice daily. In case of paediatric treatment, the dose may be reduced according to age and/or body weight.

A "therapeutically effective amount" of a compound as used herein means an amount sufficient to cure, alleviate or partially arrest the clinical manifestations of a given disease and its complications in a therapeutic intervention comprising the administration of said compound. An amount adequate to accomplish this is defined as "therapeutically effective amount". The term also includes amounts sufficient to cure, alleviate or partially arrest the clinical manifestations of a given disease and its complications in a treatment comprising the administration of said compound. Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. Nonetheless, prophylactic (preventive) and therapeutic (curative) treatment are two separate aspect of the invention. The patient to be treated is preferably a mammal, in particular a human being.

In one embodiment, the invention relates to the use of Compound I for the manufacture of a composition for oral administration for the treatment of a disease selected from mood disorders; major depressive disorder; general anxiety disorder; panic disorder; post traumatic stress disorder; depression associated with cognitive deficits, Alzheimer's disease or anxiety; depression with residual symptoms; chronic pain; eating disorder or abuse, wherein said composition is adapted so that Compound I is not released in the stomach. In one embodiment, said composition is a tablet or a multiparticulate composition coated with a pH sensitive coating, such as an enteric coating. In one embodiment, said composition, and in particular said tablet or multiparticulate composition, is adapted so that release of Compound I takes place in the small intestine.

In one embodiment, the invention relates to Compound I for use in the treatment of a disease selected from mood disorders; major depressive disorder; general anxiety disorder; panic disorder; post traumatic stress disorder; depression associated with cognitive deficits, Alzheimer's disease or anxiety; depression with residual symptoms; chronic pain; eating disorder or abuse, wherein Compound I is in a pharmaceutical composition for oral administration adapted so that Compound I is not released in the stomach. In one embodiment, said composition, and in particular said tablet or multiparticulate composition is a tablet of a multiparticulate composition coated with a pH sensitive coating, such as an enteric coating. In one embodiment, said composition, and in particular said tablet or multiparticulate composition, is adapted so that release of Compound I takes place in the small intestine.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law), regardless of any separately provided incorporation of particular documents made elsewhere herein.

The use of the terms "a" and "an" and "the" and similar referents in the context of describing the invention are to be construed to cover both the singular and the plural, unless otherwise indicated herein or clearly contradicted by context. For example, the phrase "the compound" is to be understood as referring to various "compounds" of the invention or particular described aspect, unless otherwise indicated.

Unless otherwise indicated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or aspect of the invention using terms such as "comprising", "having," "including," or "containing" with reference to an element or elements is intended to provide support for a similar aspect or aspect of the invention that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

### Example 1. Administration forms, plasma concentration-time profile and adverse events

1-[2-(2,4-dimethyl-phenylsulfanyl)-phenyl]piperazine hydrobromide (Compound I HBr) was administered to healthy volunteers in a 5-way crossover study investigating the absorption profile and the type and severity of adverse events. 23 subject (12 men and 11 women) participated in the trial; due to drop-outs however, not all 23 subjects received all five treatments - see below for details.

| Regimen | |
|---|---|
| A | ^{99m}Tc-DTPA labelled water at t=0 hours and ¹¹¹In-DTPA labelled water at t=6 hours |
| B | 20 mg Compound I HBr administered as a single oral dose of two 10 mg IR tablets with ^{99m}Tc-DTPA labelled water (t=0 hours) and followed 6 hours post-dose (t=6 hours) by ¹¹¹In-DTPA labelled water |
| C | 9 mg Compound I HBr as an iv infusion over 6 hours |
| D | 20 mg Compound I HBr solution administered via the Enterion^{TM} capsule to the proximal small bowel with a ^{99m}Tc-DTPA labelled drink |
| E | 20 mg Compound I HBr solution administered via the Enterion™ capsule to the distal small bowel with a ^{99m}Tc-DTPA labelled drink |

The proximal small bowel is approximately the first meter of the jejunum, and the distal small bowel is approximately the last meter of the ileum, including terminal ileum up to the ileo-caecal junction.

The Enterion™ capsule allows targeted delivery of an active substance into any region of the GI tract. The capsule contains a drug reservoir and a separate compartment for a radioactive tracer. The floor of the reservoir acts a piston which is held back against a spring by a polymer filament. By means of gamma scintigraphy (an imaging technique) the exact position of the capsule in the GI tract can be determined real-time. When the capsule has reached its desired position in the GI tract, a magnetic field is applied which causes an antenna in the capsule all to heat up. The heated antenna is in contact with the polymer filament which softens and eventually breaks which sets the piston (reservoir floor) free and rapidly releases the drug. The Enterion™ capsule is available from Partner Tech, UK.

Blood samples were obtained at regular intervals 240 hours post-dosing of the IR tablet, post-start of the infusion and post-activation of the Enterion™ capsule. These samples were later analysed to obtain the plasma concentration-time profile for Compound I HBr. Additionally, adverse events were recorded. Food (soup) was provided 4 hours post-dose and dinner 10 hours post-dose.

All 23 subjects received treatments A and B; 22 subjects received treatment C; 20 subjects received treatment D; and 19 received treatment E.

The table below summarises the adverse events recorded during the course of the trial.

| Adverse Event | Regimen B (no of subjects) | Regimen C (no of subjects) | Regimen D (no of subjects) | Regimen E (no of subjects) |
|---|---|---|---|---|
| Nausea | 9 | 1 | 3 | 2 |
| Vomiting | 2 | | 1 | |
| Retching | 1 | | | |
| Loose stool / diarrhoea | 6 | 1 | 2 | 3 |
| Abdominal pain | 2 | | 1 | 1 |
| Lightheadedness / postural dizziness (no BP drop) | 5 | | 1 | |
| Postural hypotension | | 2 | 1 | 3 |
| Headache | 3 | 2 | 1 | |
| Fatigue | 2 | | | |
| Rash | | | | 1 |
| Pruritis | | | 1 | |
| Abnormal liver function tests | | 1 | | |

The above data clearly indicates that the number of adverse events, and in particular GI tract related adverse events, depends on the administration form, and more specifically on where Compound I is released. The most adverse events occur in regimen B, i.e. administration of Compound I in an IR tablet. A markedly lower number of adverse events occur in regimen C, D and E, i.e. when Compound I administered by i.v. infusion, and released in the proximal or distal small bowl, respectively. From these data it can be fairly concluded that administration of Compound I which avoids release in the stomach, such as iv administration or with release of Compound I in the small intestine (e.g. enteric coated tablet or enteric coated multiparticulate composition) is associated with less adverse events.

The plasma concentration time profiles are depicted in Figure 1. From these data it is clear that the plasma profile of Compound I is virtually independent on whether Compound I is administered in an IR tablet or released in the small intestine. Intravenous infusion of Compound I also gives rise to a similar plasma profile.

### Example 2. Modified release swelling matrix tablet based on a hydrocolloid matrix of HPMC (hydroxypropylmethylcellulose).

| **Substance** | **mg** | **%** |
|---|---|---|
| Compound I | 30 | 12.0 |
| Avicel PH200 (microcrystalline cellulose) | 118.7 | 47.5 |
| Metolose 90SH100SR (hydroxypropylmethylcellulose) | 70 | 28.0 |
| Mannitol | 30 | 12.0 |
| Magnesium stearate | 1.3 | 0.5 |
| | 250 | 100 |

240 g Compound I is mixed with 950 g Avicel PH200 (microcrystalline cellulose), 560 Metolose 90SH100SR and 240 g Mannitol for 3 minutes in a Turbula mixer. Subsequently 10.4 g magnesium stearate is admixed for 0.5 minutes in the Turbula mixer. Tablets of 8 mm in diameter with compound shape are compressed on a single punch tableting machine Korsch EK0. Tablet hardness: 85 N.

### Example 3. Modified release swelling matrix tablet based on a hydrocolloid matrix of HPMC (hydroxypropylmethylcellulose) comprising enteric coating (acrylic polymer)

The tablets of Example 2 are enteric coated with a film coating formulation according to the following composition.

| Substance | % |
|---|---|
| Eudragit L30D-55 (methacrylic acid-ethyl acrylate copolymer (1:1) in aqueous latex suspension) | 40 |
| Purified water | 52 |
| Triethyl citrate | 1.8 |
| Simethicone antifoam emulsion | 0.2 |
| Talc (fine) | 6 |

The coating suspension is prepared by mixing triethylcitrate, antifoam emulsion and purified water in Ultra Turrax apparatus at 9500 rpm for 10 minutes. After 1 minute the talc is added. The Eudragit suspension is added afterwards under gentle stirring and the suspension is finally passed through sieve 0.3 mm. 1.2 kg of the tablets is coated in a rotating perforated vessel (CombuLab). Inlet air temperature: 40 °C. 500 m³/h. Outlet air temperature: 33 °C. Spray rate 8-10 g/min. Atomizing pressure: 2 Bar. The coating is continued until a weight increase of 8% was achieved after approx. 1 hour.

### Example 4. Multiparticulate composition based on enteric coated pellets.

Compound I is layered on placebo pellets (MCC spheres-Celphere CP-203) as a drug suspension. The composition of the drug layered pellets is according to the following.

| Substance | mg | % |
|---|---|---|
| Compound I | 30 | 8.8 |
| Celphere-203 (pellets composed of microcrystalline cellulose, diameter 250 µm) | 300 | 88.2 |
| PVP 30 (polyvinylpyrrolidone) | 10 | 2.9 |
| | 340 | 100 |

An aqueous suspension of Compound I with PVP 30 as binder with the ratio water/drug/PVP of 85/11.25/3.75 is sprayed on 1.76 kg pellets in a fluid bed Aeromatic MP-1 using a Wurster insert. Inlet air temperature: 60-70 °C. 80 m³/h. Outlet air temperature: 35-45 °C. Spray rate 10-15 g/min for 2 hours. Atomizing pressure: 1 Bar.

The drug layered pellets are subsequently coated with the enteric coating of Example 3. 2 kg of pellets are loaded into the fluid bed Aeromatic MP-1 using a Wurster insert. Inlet air temperature: 60 °C. 90 m³/h. Outlet air temperature: 30 °C. Spray rate 10-15 g/min. Atomizing pressure: 1 Bar. The coating is continued until a weight increase of 20% is achieved after approx. 2.5 hours. The enteric coated pellets are filled into hard gelatin capsules.

### Example 5. 30mg delayed release composition based on enteric coated tablet cores.

Granules were first made by blending, granulating and drying in a Fluid bed Aeromatic MP-1. An aqueous suspension of 6 w/w % Klucel EXF was sprayed on 26500 g of blend (Compoun I HBr, Mannitol 50c, Avicel PH 101 and Soduim starch glycolate (type A)).

| **Substance** | **mg** | **%** |
|---|---|---|
| Compound I HBr | 38.13 | 12.71 |
| Mannitol 50c | 195.87 | 65.29 |
| Avicel PH 101 (microcrystalline cellulose, particle size ∼70µm) | 45.00 | 15 |
| Primojel (Sodium starch glycolate) | 9 | 3 |
| Klucel EXF (hydroxypropylcellulose) | 9 | 3 |
| Magnesium stearate | 3 | 1 |

Inlet air temperature: 60 °C, 500 m³/h. Outlet air temperature: 26 °C. Spray rate 500-700 g/min. Atomizing pressure: 3 Bar. Next the granules were dried to a relative humidity of the granulate of 25-55 % RH: Inlet air temperature: 60 °C, 500 m³/h. The resulting granules were then passed through a 1.5748 mm screen and blended with 1500 g Avicel PH 101 and 900 g Klucel EXF in a bohle blender (8min, 7rotaions /minute). Then 300 g magnesium stearate was added and the mixture was blended for 3 minutes, 7 rotations/ minute. Using a Korsch press fitted with 9 mm punches the blend was compressed into tablet cores. Next the tablets were enteric coated with a film coating formulation according to the following composition.

| Substance | % |
|---|---|
| Acryl-EZE ((methacrylic acid-ethyl acrylate copolymer (1:1) in aqueous latex suspension) | 40 |
| Purified water | 52 |
| Triethyl citrate | 1.8 |
| Simethicone antifoam emulsion | 0.2 |

The coating suspension was prepared by mixing triethylcitrate, antifoam emulsion and purified water in mixing vessel. The Acryl-EZE suspension was added afterwards under gentle stirring and the suspension was finally passed through sieve 0.3 mm. 1.2 kg of the tablets was coated in a rotating perforated vessel (CombuLab). Inlet air temperature: 40 °C. 500 m³/h. Outlet air temperature: 33 °C. Spray rate 8-12 g/min. Atomizing pressure: 2 Bar. The coating was continued until a weight increase of 12% was achieved after approx. 90 minutes.

When exposed to the dissolution test described above, the coated tablets had the following dissolution profile.

| | Stage 1 | Stage 2 | | | | |
|---|---|---|---|---|---|---|
| **Time** | 2 hours | +10 min | +20 min | +30 min | +45 min | + 60 min |
| **% Release** | 0 | 0 | 47 | 77 | 88 | 91 |

Compound I was analysed on an HLPC system fitted with a Symmetry Shield RP18, 2.1x20 mm ID, 3.5 µm column. The mobile phase was 20 mM acetate buffer pH 4.8/acetonitrile (75/25). Flow rate 2.0 ml/min and detection in a UV detector at 226 nm.

## Claims

1. A pharmaceutical composition for oral administration comprising the compound 1-[2-(2,4-dimethyl-phenylsulfanyl)-phenyl]piperazine and pharmaceutically acceptable acid addition salts thereof, wherein said composition is adapted so that said compound is not released in the stomach.

2. The composition according to claim 1, wherein said composition is a sustained release composition.

3. The composition according to claim 1, wherein said composition is a delayed release composition.

4. The composition of claim 3, wherein said composition comprises a pH-sensitive coating.

5. The composition of claim 4, which composition is an enteric coated tablet.

6. The composition according to claim 4, which composition is a multiparticulate composition in which substantially each particle is enteric coated.

7. The composition according to claim 1, which composition is a tablet comprising the compound 1-[2-(2,4-dimethyl-phenylsulfanyl)-phenyl]piperazine and pharmaceutically acceptable acid addition salts thereof, mannitiol, microcrystalline cellulose, sodium starch glycolate, hydroxypropyl cellulose and magnesium stearate, which tablet is coated with methacrylic acid ethyl acrylate (1:1) copolymer.

8. The composition according to any of claims 1-7, wherein said compound is 1-[2-(2,4-dimethyl-phenylsulfanyl)-phenyl]piperazine HBr in an amount of 1-50 mg.

9. The compound 1-[2-(2,4-dimethyl-phenylsulfanyl)-phenyl]piperazine and pharmaceutically acceptable acid addition salts thereof for use in the treatment of a disease selected from mood disorders; major depressive disorder; general anxiety disorder; panic disorder; post traumatic stress disorder; depression associated with cognitive deficits, Alzheimer's disease or anxiety; depression with residual symptoms; chronic pain; eating disorder or abuse, wherein said compound is in a pharmaceutical composition for oral administration adapted so that said compound is not released in the stomach.

10. The compound according to claim 9, wherein said composition is a tablet or a multiparticulate composition coated with a pH sensitive coating

11. The compound according to claim 10, wherein said pH sensitive coating is an enteric coating.

12. The compound according to claim 9, wherein said composition is adapted so that release of Compound I takes place in the small intestine.

13. The compound according to any of claims 9-12, which compound is 1-[2-(2,4-dimethyl-phenylsulfanyl)-phenyl]piperazine HBr I an amount of 1-50 mg.

## Patentansprüche

1. Pharmazeutische Zusammensetzung zur oralen Verabreichung, enthaltend die Verbindung 1-[2-(Dimethylphenylsulfanyl)-phenyl]piperazin und pharmazeutisch akzeptable Säureadditionssalze derselben, wobei diese Zusammensetzung so angepasst ist, dass die Verbindung nicht im Magen freigesetzt wird.

2. Zusammensetzung nach Anspruch 1, wobei die Zusammensetzung eine Zusammensetzung mit retardierter Freisetzung ist.

3. Zusammensetzung nach Anspruch 1, wobei die Zusammensetzung eine Zusammensetzung mit verzögerter Freisetzung ist.

4. Zusammensetzung nach Anspruch 3, wobei die Zusammensetzung eine pH-empfindliche Beschichtung aufweist.

5. Zusammensetzung nach Anspruch 4, welche Zusammensetzung eine enterisch beschichtete Tablette ist.

6. Zusammensetzung nach Anspruch 4, welche Zusammensetzung eine multipartikuläre Zusammensetzung ist, bei welcher im Wesentlichen jedes Partikel enterisch beschichtet ist.

7. Zusammensetzung nach Anspruch 1, welche Zusammensetzung eine Tablette ist, welche die Verbindung 1-[2-(Dimethylphenylsulfanyl)-phenyl]piperazin und pharmazeutisch akzeptable Säureadditionssalze derselben, Mannitiol, mikrokristalline Zellulose, Natriumstärkeglykolat, Hydroxypropylzellulose und Magnesiumstearat enthält, welche Tablette mit Methacrylsäureethylacrylat(1:1)-Copolymer beschichtet ist.

8. Zusammensetzung nach einem der Ansprüche 1-7, bei welcher die Verbindung 1-[2-(2,4-Dimethylphenylsulfanyl)-phenyl]piperazin-HBr in einer Menge von 1-50 mg ist.

9. Verbindung 1-[2-(2,4-Dimethylphenylsulfanyl)-phenyl]piperazin und pharmazeutisch akzeptable Säureadditionssalze derselben zur Verwendung bei der Behandlung einer Erkrankung, die ausgewählt ist aus affektiven Störungen; schwerer depressiver Störung; allgemeiner Angststörung; Panikstörung; posttraumatischer Belastungsstörung; Depression in Verbindung mit kognitiven Beeinträchtigungen, Alzheimer-Erkrankung oder Angstzuständen; Depression mit Restsymptomen; chronischem Schmerz; Essstörung oder -missbrauch, wobei die Verbindung in einer pharmazeutischen Zusammensetzung zur oralen Verabreichung ist, die so angepasst ist, dass die Verbindung nicht im Magen freigesetzt wird.

10. Verbindung nach Anspruch 9, wobei die Verbindung eine Tablette oder eine multipartikuläre Zusammensetzung ist, die mit einer pH-empfindlichen Beschichtung beschichtet ist.

11. Verbindung nach Anspruch 10, wobei die pH-empfindliche Beschichtung eine enterische Beschichtung ist.

12. Verbindung nach Anspruch 9, wobei die Zusammensetzung so angepasst ist, dass die Freisetzung der Verbindung I im Dünndarm stattfindet.

13. Verbindung nach einem der Ansprüche 9-12, welche Verbindung 1-[2-(2,4-Dimethylphenylsulfanyl)-phenyl]piperazin-HBr I in einer Menge von 1-50 mg ist.

## Revendications

1. Composition pharmaceutique pour administration orale comprenant le composé 1-[2-(2,4-diméthyl-phénylsulfanyl)-phényl]pipérazine et des sels d'addition d'acide de celui-ci acceptables d'un point de vue pharmaceutique, dans laquelle ladite composition est conçue de sorte que ledit composé n'est pas libéré dans l'estomac.

2. Composition selon la revendication 1, dans laquelle ladite composition est une composition à libération prolongée.

3. Composition selon la revendication 1, dans laquelle ladite composition est une composition à libération retardée.

4. Composition selon la revendication 3, dans laquelle ladite composition comprend un revêtement sensible au pH.

5. Composition selon la revendication 4, laquelle composition est un comprimé entéro-soluble.

6. Composition selon la revendication 4, dans laquelle la composition est une composition multiparticulaire dans laquelle sensiblement chaque particule est entéro-soluble.

7. Composition selon la revendication 1, laquelle composition est un comprimé comprenant le composé 1-[2-(2,4-diméthyl-phénylsulfanyl)-phényl]pipérazine et des sels d'addition d'acide de celui-ci acceptables d'un point de vue pharmaceutique, du mannitiol, de la cellulose microcristalline, du glycolate d'amidon sodique, de l'hydroxypropyl cellulose et du stéarate de magnésium, lequel comprimé est revêtu de copolymère d'acide méthacrylique - acrylate d'éthyle (1:1).

8. Composition selon l'une quelconque des revendications 1 à 7, dans lequel ledit composé est du 1-[2-(2,4-diméthyl-phénylsulfanyl)-phényl]pipérazine HBr en une quantité de 1 à 50 mg.

9. Composé de 1-[2-(2,4-diméthyl-phénylsulfanyl)-phényl]pipérazine et sels d'addition d'acide de celui-ci acceptables d'un point de vue pharmaceutique pour utilisation dans le traitement d'une maladie sélectionnée à partir des troubles de l'humeur ; état dépressif majeur ; trouble général de l'anxiété ; trouble panique ; état de stress post-traumatique ; dépression associée à des déficits cognitifs, maladie d'Alzheimer ou anxiété ; dépression avec symptômes résiduels ; douleur chronique ; trouble de l'alimentation ou abus, dans lequel ledit composé est dans une composition pharmaceutique pour administration orale conçue de sorte que ledit composé n'est pas libéré dans l'estomac.

10. Composé selon la revendication 9, dans lequel ladite composition est un comprimé ou une composition multiparticulaire revêtue d'un revêtement sensible au pH.

11. Composé selon la revendication 10, dans laquelle ledit revêtement sensible au pH est un revêtement entérique.

12. Composé selon la revendication 9, dans laquelle ladite composition est conçue de sorte que la libération du Composé I a lieu dans l'intestin grêle.

13. Composé selon l'une quelconque des revendications 9 à 12, lequel composé est du 1-[2-(2,4-diméthyl-phénylsulfanyl)-phényl]pipérazine HBr I en une quantité de 1 à 50 mg.
